# EUROPÄISCHE PATENTSCHRIFT

(11) **EP 0 804 910 B1**
(45) Veröffentlichungstag und Bekanntmachung des Hinweises auf die Patenterteilung: **17.10.2001**
(21) Anmeldenummer: 97102431.0
(22) Anmeldetag: 14.02.1997
(51) Int. Cl.: A61F 2/52

(54) **Brustprothese**
Breast prosthesis
Prothèse mammaire

(30) Priorität: 02.05.1996 DE 9607969 U
(43) Veröffentlichungstag der Anmeldung: 05.11.1997
(73) Patentinhaber: AMOENA Medizin-Orthopädie-Technik GmbH, D-83064 Raubling (DE)
(72) Erfinder: Mulligan, Elisabeth, 83083 Riedering-Söllhuben (DE); Wild, Helmut, 83115 Neubeuern (DE)
(74) Vertreter: Laufhütte, Dieter, Dr.-Ing.

(56) Entgegenhaltungen:
- EP-A- 0 320 590
- EP-A- 0 542 119
- DE-U- 9 315 935

## Beschreibung

Die Erfindung betrifft eine Brustprothese nach dem Oberbegriff des Anspruchs 1.

Eine derartige Brustprothese ist aus der EP-0 320 590 A1 und der DE 9 315 935 U bekannt.

Ein besonderes Problem bei derartigen Brustprothesen besteht darin, diese gut und möglichst unverrutschbar an der Brust der Trägerin zu befestigen.

Aus der DE-91 24 88 ist es bekannt, eine Prothese auf ihrer Rückseite in ihrem Randbereich mit einer Klebeschicht zu versehen.

Aus der DE 9 315 935 U ist es bekannt, eine derartige Brustprothese auf ihrem auf der Rückseite der Prothese liegenden Randbereich, der aus der harten äußeren Schicht gebildet ist, mit einer Haftschicht zu versehen.

Es ist jedoch bei derartigen Klebeschichten festzustellen, daß die Haftung und auch die Haftdauer am Körper mit zunehmender Härte bzw. Festigkeit des verwendeten Prothesenmaterials abnimmt, da auf die Prothese einwirkende Zug- bzw. Drehkräfte unmittelbar auf eine solche Klebeschicht übertragen werden.

Andererseits ist es ungünstig, eine derartige Klebeschicht auf einem zu weichen Material vorzusehen, da in diesem Fall Probleme bei der Aufrechterhaltung der gewünschten Form der Prothese auftreten, und ferner beim Abnehmen der Prothese auftretende Zug-und Schälkräfte bei einem zu weichen Material nicht wirksam aufgefangen werden können.

Aufgabe der Erfindung ist es daher, eine Brustprothese der eingangs angegebenen Art derart weiterzubilden, daß sie sich in einfacher Weise lösbar unmittelbar auf der Haut einer Trägerin befestigen läßt, wobei die Haftdauer der Prothese erhöht werden soll.

Erfindungsgemäß wird diese Aufgabe bei einer Brustprothese der eingangs angegebenen Art dadurch gelöst, daß diese auf ihrer Rückseite in ihrem Randbereich mit einer dauernd klebrig bleibenden Haftschicht versehen ist, wobei ein erster Bereich der Haftschicht auf dem äußeren Prothesenkörper, und ein zweiter Bereich der Haftschicht auf dem inneren Prothesenkörper angeordnet ist.

Durch die Anordnung der Haftschicht in der Weise, daß ein erster Bereich mit dem härteren, äußeren Prothesenkörper, und ein zweiter Bereich mit dem weicheren, inneren Prothesenkörper in Verbindung steht, können durch Bewegungen der Trägerin verursachte Kräfte in vorteilhafter Weise von der Brustprothese als ganzes aufgefangen werden, so daß keine übermäßige Belastung der Haftschicht auftritt. Hierdurch ist die Langlebigkeit der Haftschicht bzw. eine lange Haftdauer gewährleistet.

Der äußere Prothesenkörper ist in der Lage, um den Bereich, in welchem er der Haftschicht verbunden ist, in gewissem Ausmaß Drehbewegungen auszuführen, da der unter dem äußeren Prothesenkörper liegende, wesentlich weichere innere Prothesenkörper stärker elastisch deformierbar ist. Ferner ist der innere Prothesenkörper durch seine Elastizität bzw. Weichheit in der Lage, sich an Unregelmäßigkeiten der Hautoberfläche der Trägerin anzupassen, wodurch eine verbesserte Haftreibung erzielbar ist.

Bevorzugt beträgt der Flächenanteil des ersten Bereiches etwa 1/4 bis 1/3 der Gesamtfläche der Haftschicht.

Zweckmäßigerweise besteht die Schicht aus einzelnen, im Abstand voneinander angeordneten klebrigen Abschnitten.

In vorteilhafter Weise ist die dauernd klebrig bleibende Schicht umlaufend ausgebildet.

Nach einer vorteilhaften Ausgestaltung ist vorgesehen, daß die Prothese in ihrem Randbereich auf ihrer Rückseite mit einer umlaufenden Nut versehen ist, in der die Klebeschicht eingebettet ist.

Zweckmäßigerweise ist die Klebeschicht durch eine aufgelegte Abdeckfolie geschützt. Diese wird vor dem Anlegen der Prothese abgezogen.

Ein Ausführungsbeispiel der Erfindung wird nachstehend anhand der Zeichnungen näher erläutert. In diesen zeigen
- Fig. 1: eine seitliche Schnittansicht einer ersten Ausführungsform der erfindungsgemäßen Brustprothese,
- Fig. 2: eine seitliche Schnittansicht einer zweiten Ausführungsform der erfindungsgemäßen Brustprothese,
- Fig. 3: eine seitliche Schnittansicht einer dritten Ausführungsform der erfindungsgemäßen Brustprothese, und
- Fig. 4: die Rückseite einer mit einer umlaufenden Klebesicht versehenen Brustprothese.

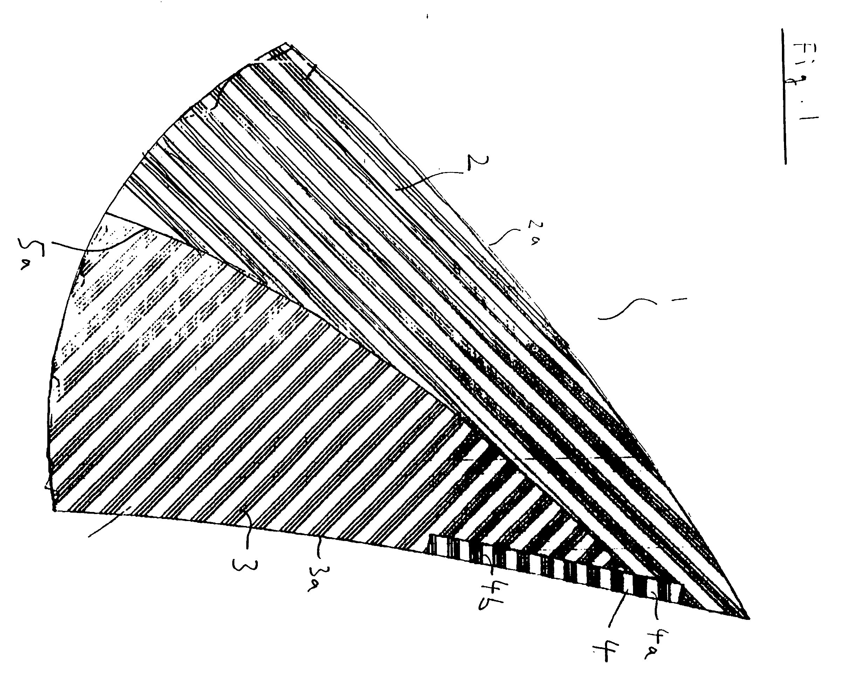

Die in Fig. 1 dargestellte Brustprothese besteht aus einem äußeren Körper 2, beispielsweise aus einer weich eingestellten additionsvernetzenden Zweikomponenten-Silikonkautschuk-Masse, und einem den größten Teil der Prothesenrückseite bildenden Körper 3, der aus einer weicheren Masse mit gelartiger Konsistenz, beispielsweise aus additionsvernetzendem Zweikomponenten-Silikonkautschuk, besteht. Die beiden Körper 2, 3 sind auf ihren Außenseiten von Kunststoff-Folien 2a, 3a, beispielsweise Polyurethanfolien, eingefaßt, und voneinander durch eine Zwischenfolie 5a getrennt.

Auf der Rückseite der Brustprothese 1 ist eine Haftschicht 4 vorgesehen. Ein erster Bereich 4a dieser Haftschicht steht hierbei mit dem äußeren, der Brustform nachgebildeten Prothesenkörper 2 in Verbindung, während einer zweiter Bereich 4b der Haftschicht auf dem inneren Körper 3 anliegt.

Durch diese Anordnung der Haftschicht ist eine optimale Anbringung der Brustprothese an der Haut der Trägerin gewährleistet.

Durch Bewegungen der Trägerin verursachte, auf die Brustprothese wirkende Kräfte können durch die gewissermaßen "gelenkige" Ausbildung der Brustprothese von der Haftschicht aufgefangen werden. Diese "Gelenkigkeit" wird dadurch erreicht, daß durch die sehr weiche, gelartige Konsistenz des Prothesenkörpers 3 eine federnde Drehbewegung des äußeren Prothesenkörpers 2 mit dem Bereich 4a der Haftschicht als Drehzentrum möglich ist. Hierdurch ist gewährleistet, daß insbesondere der Bereich 4b der Haftschicht nur sehr wenig belastet wird, wodurch die Haftung am Körper der Trägerin nicht beeinträchtigt wird. Somit ist die Haftdauer eines erfindungsgemäßen Prothesenkörpers am Körper der Trägerin erhöht.

Vorteilhafterweise beträgt der Anteil des Bereiches der Haftschicht, der auf dem härteren und festeren Silikon angeordnet ist, etwa 1/4 bis 1/3 der Gesamthaftschichtfläche. Hierdurch ist gewährleistet, daß beim Abnehmen der Prothese auftretende Zug- und Schälkräfte über diese Schicht aufgefangen werden können.

Gleichzeitig ermöglicht der die gelartige Konsistenz aufweisende zweite Prothesenkörper 3 eine optimale Anpassung der Haftschicht 4 an Unebenheiten der Haut der Trägerin, beispielsweise durch eine Operation verursachte Narben.

Wie in Fig. 1 ferner zu erkennen, ist die Haftschicht 4 in einer auf der Innenseite des Prothesenkörpers ausgebildeten Nut vorgesehen, so daß die Außenseite der Haftschicht 4 mit der Rückseite der Prothese bündig verläuft. Durch diese Maßnahme ist sowohl der Tragekomfort für die Trägerin erhöht, als auch eine verbesserte Haftung der Haftschicht erzielbar.

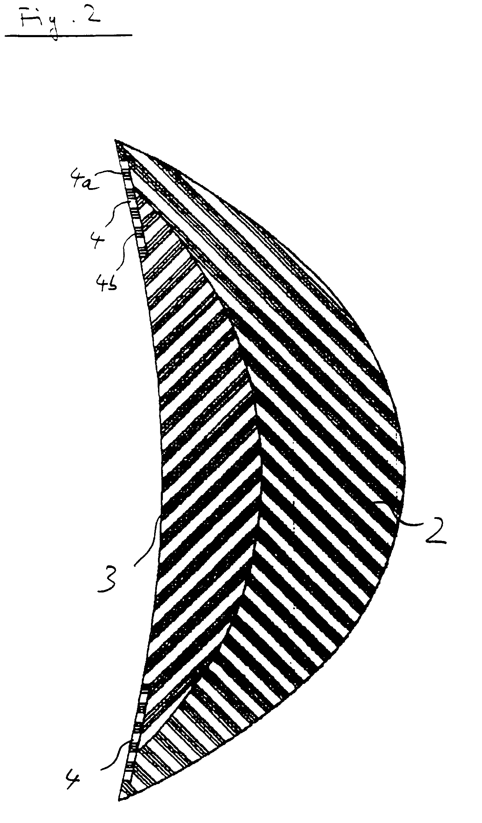

In Fig. 2 ist eine weitere Ausführungsform der erfindungsgemäßen Brustprothese dargestellt. Im Unterschied zum Ausführungsbeispiel der Fig. 1 ist hier die Haftschicht 4 umlaufend ausgebildet.

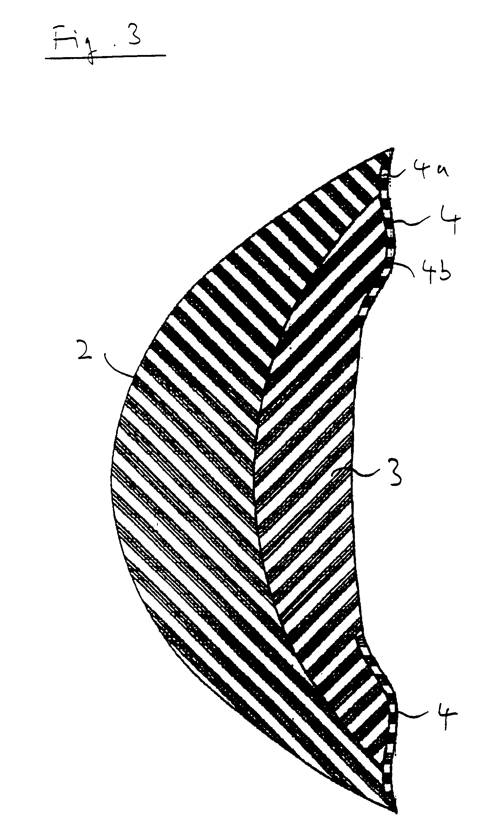

Fig. 3 zeigt eine weitere Ausführungsform der erfindungsgemäßen Brustprothese. Man erkennt, daß der Außenbereich des inneren Prothesenkörpers 3 wellenartig nach außen vorstehend ausgebildet ist. Der innere Bereich 4b der Haftschicht 4 weist eine entsprechende wellenartige Form auf. Durch diesen wellenartig vorstehenden Bereich 4b ist eine erhöhte Beweglichkeit und Anpassungsfähigkeit dieses Prothesenbereiches gewährleistet, wodurch dieser Prothesenbereich in besonders vorteilhafter Weise an ein Narbengebiet der Trägerin angepaßt werden kann.

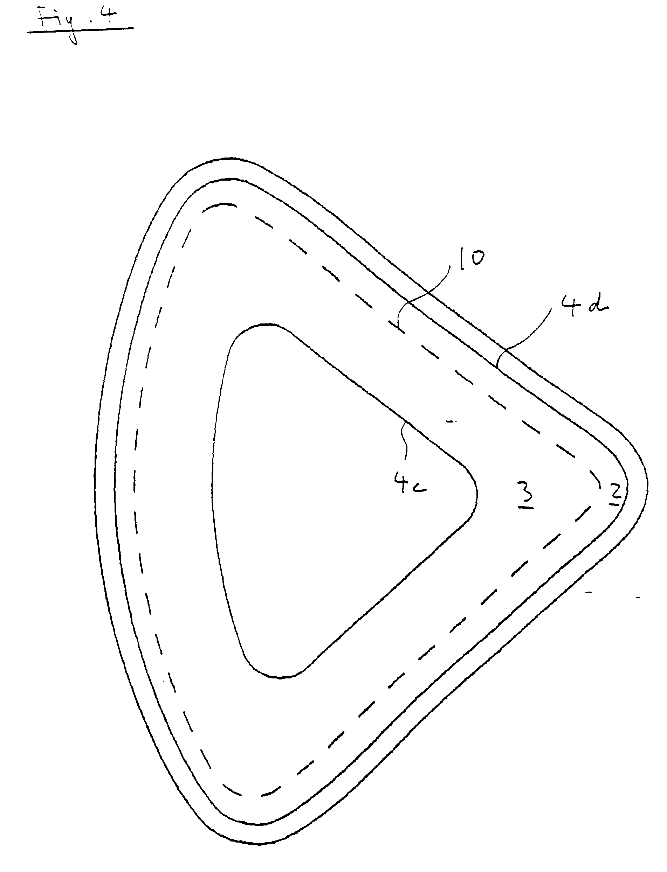

Schließlich sind in Fig. 4 die bevorzugten Dimensionierungen der Haftschicht 4 auf der Rückseite der Prothesenkörper 2, 3 schematisch dargestellt. Hierbei stellen die durchgezogenen Linien 4c und 4d den inneren bzw. äußeren Rand der Haftschicht dar. Die gestrichelte Linie 10 markiert die Grenze zwischen äußeren und inneren Prothesenkörpern 2, 3.

## Patentansprüche

1. Brustprothese, bestehend aus zwei jeweils in Kunststoff-Folien eingeschweißten schalenförmigen Körpern (2, 3) aus Silikonmasse unterschiedlicher Weichheit, wobei der äußere, der Brustform nachgebildete Körper (2) eine Härte besitzt, die der weich-elastischen Nachgiebigkeit des natürlichen Brustgewebes angeglichen ist, und der innere Körper (3) eine weichere, gelartige Konsistenz aufweist, und
die Prothese auf ihrer Rückseite in ihrem Randbereich mit einer dauernd klebrig bleibenden Haftschicht (4) versehen ist, wobei ein erster Bereich (4a) der Haftschicht (4) auf dem äußeren Prothesenkörper (2) angeordnet ist, **dadurch gekennzeichnet, daß** ein zweiter Bereich (4b) der Haftschicht (4) auf dem inneren Körper (3) angeordnet ist.

2. Brustprothese nach Anspruch 1, **dadurch gekennzeichnet, daß** die dauernd klebrig bleibende Schicht aus im Abstand voneinander angeordneten klebrigen Abschnitten besteht.

3. Brustprothese nach Anspruch 1, **dadurch gekennzeichnet, daß** die dauernd klebrig bleibende Haftschicht umlaufend ausgebildet ist.

4. Brustprothese nach einem der Ansprüche 1 bis 3, **dadurch gekennzeichnet, daß** die Prothese in ihrem Randbereich auf ihrer Rückseite mit einer umlaufenden Nut versehen ist, in der die dauernd klebrig bleibende Schicht (4) eingebettet ist.

5. Brustprothese nach einem der Ansprüche 1 bis 4, **dadurch gekennzeichnet, daß** der Flächenanteil des ersten Bereiches (4a) der Haftschicht etwa 1/4 bis 1/3 der Gesamtfläche der Haftschicht (4) beträgt.

## Claims

1. Breast prosthesis, consisting of two shell-shaped bodies (2, 3) made of silicone composition of different thickness and each welded into plastic films, the outer body (2) simulating the breast shape and having a hardness matching the softly resilient compliance of the natural breast tissue, and the inner body (3) having a softer, gel-like consistency, and the prosthesis being provided on its reverse, in its edge area, with a permanently tacky adhesive layer (4), a first area (4a) of the adhesive layer (4) being arranged on the outer prosthesis body (2), **characterized in that** a second area (4b) of the adhesive layer (4) is arranged on the inner body (3).

2. Breast prosthesis according to Claim 1, **characterized in that** the permanently tacky layer is made up of tacky portions arranged at a distance from one another.

3. Breast prosthesis according to Claim 1, **characterized in that** the permanently tacky adhesive layer is formed around the periphery.

4. Breast prosthesis according to one of Claims 1 to 3, **characterized in that**, in its edge area on its reverse, the prosthesis is provided with a peripheral groove in which the permanently tacky layer (4) is embedded.

5. Breast prosthesis according to one of Claims 1 to 4, **characterized in that** the surface portion of the first area (4a) of the adhesive layer amounts to approximately 1/4 to 1/3 of the total surface area of the adhesive layer (4).

## Revendications

1. Prothèse mammaire constituée de deux corps en forme de coque (2, 3) soudés respectivement dans des feuilles de matière synthétique, en une masse de silicone d'une souplesse différente, où le corps extérieur (2) réalisé selon la forme du sein possède une dureté qui est adaptée à la souplesse molle et élastique du tissu naturel du sein, et le corps intérieur (3) a une consistance plus molle en forme de gel, et la prothèse est munie sur son côté arrière dans sa zone de bord d'une couche d'adhésion (4) qui reste collante en permanence, où une première zone (4a) de la couche d'adhésion (4) est disposée sur le corps de prothèse extérieur (2), **caractérisée en ce qu'**une deuxième zone (4b) de la couche d'adhésion (4) est disposée sur le corps intérieur (3).

2. Prothèse mammaire selon la revendication 1, **caractérisée en ce que** la couche qui reste collante en permanence est constituée de sections collantes disposées à une certaine distance les unes des autres.

3. Prothèse mammaire selon la revendication 1, **caractérisée en ce que** la couche d'adhésion qui reste collante en permanence est réalisée pour s'étendre tout autour.

4. Prothèse mammaire selon l'une des revendications 1 à 3, **caractérisée en ce que** la prothèse présente dans sa zone de bord sur son côté arrière une rainure s'étendant tout autour dans laquelle est noyée la couche (4) qui reste collante en permanence.

5. Prothèse mammaire selon l'une des revendications 1 à 4, **caractérisée en ce que** la part de surface de la première zone (4a) de la couche d'adhésion représente environ 1/4 à 1/3 de la face totale de la couche d'adhésion (4).
